(19) **Europäisches Patentamt / European Patent Office / Office européen des brevets**

(11) **EP 3 848 492 B1**

(12) # EUROPEAN PATENT SPECIFICATION

(45) Date of publication and mention
of the grant of the patent:
**03.06.2026 Bulletin 2026/23**

(21) Application number: **19874990.5**

(22) Date of filing: **25.10.2019**

(51) International Patent Classification (IPC):
*D04H 3/16* (2006.01)     *A41D 13/11* (2006.01)
*A61F 13/51* (2006.01)     *A61F 13/511* (2006.01)
*A61F 13/514* (2006.01)     *B32B 5/26* (2006.01)
*B32B 27/32* (2006.01)     *D04H 3/007* (2012.01)
*A61F 13/49* (2006.01)     *B32B 5/02* (2006.01)
*B32B 7/022* (2019.01)     *B32B 5/06* (2006.01)
*B32B 7/09* (2019.01)     *B32B 7/12* (2006.01)
*B32B 27/12* (2006.01)     *B32B 27/20* (2006.01)
*B32B 27/34* (2006.01)     *B32B 27/36* (2006.01)
*B32B 27/40* (2006.01)

(52) Cooperative Patent Classification (CPC):
**B32B 7/12; B32B 5/02; B32B 5/022; B32B 5/024;
B32B 5/026; B32B 5/06; B32B 5/26; B32B 7/022;
B32B 7/09; B32B 27/12; B32B 27/205;
B32B 27/32; B32B 27/34; B32B 27/36;
B32B 27/40;** (Cont.)

(86) International application number:
**PCT/JP2019/041999**

(87) International publication number:
**WO 2020/085502 (30.04.2020 Gazette 2020/18)**

(54) **MULTILAYER NONWOVEN FABRIC, STRETCHABLE MULTILAYER NONWOVEN FABRIC, FIBER PRODUCT, ABSORBENT ARTICLE, AND SANITARY MASK**

MEHRSCHICHTIGER VLIESSTOFF, STRECKBARER MEHRSCHICHTIGER VLIESSTOFF, FASERPRODUKT, ABSORBIERENDER ARTIKEL UND HYGIENEMASKE

TISSU NON TISSÉ MULTICOUCHE, TISSU NON TISSÉ MULTICOUCHE EXTENSIBLE, PRODUIT EN FIBRE, ARTICLE ABSORBANT ET MASQUE HYGIÉNIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.10.2018 JP 2018201247**

(43) Date of publication of application:
**14.07.2021 Bulletin 2021/28**

(73) Proprietor: **Mitsui Chemicals Asahi Life Materials
Co., Ltd.
Tokyo 104-0028 (JP)**

(72) Inventors:
• **TAKAKU, Shouichi
Nagoya-shi, Aichi 457-8522 (JP)**

• **SHIMADA, Koichi
Sodegaura-shi, Chiba 299-0265 (JP)**
• **MOTOMURA, Shigeyuki
Sodegaura-shi, Chiba 299-0265 (JP)**

(74) Representative: **Hoffmann Eitle
Patent- und Rechtsanwälte PartmbB
Arabellastraße 30
81925 München (DE)**

(56) References cited:
EP-A1- 3 779 016       WO-A1-2007/138733
WO-A1-2016/143834       WO-A1-2019/188134
JP-A- 2016 504 060       JP-A- 2017 197 890
JP-A- 2017 197 890       US-A1- 2006 246 803
US-A1- 2008 119 103       US-A1- 2014 127 459
US-A1- 2017 000 659       US-A1- 2018 038 025

EP 3 848 492 B1

(52) Cooperative Patent Classification (CPC): (Cont.)
**D04H 3/007; D04H 3/16;** A61F 13/511;
A61F 13/514; B32B 2250/20; B32B 2250/40;
B32B 2262/0215; B32B 2262/0223; B32B 2262/023;
B32B 2262/0238; B32B 2262/0246;

B32B 2262/0253; B32B 2262/0261;
B32B 2262/0276; B32B 2262/0292; B32B 2262/12;
B32B 2274/00; B32B 2307/50; B32B 2307/51;
B32B 2307/54; B32B 2307/718; B32B 2307/724;
B32B 2307/728; B32B 2535/00; B32B 2555/02

**Description**

Technical Field

The present invention relates to a multilayer nonwoven fabric, a stretchable multilayer nonwoven fabric, a fiber product, an absorbent article, and a sanitary mask.

Background Art

In recent years, nonwoven fabrics are widely used in various applications because of their excellent air breathability and flexibility. For that reason, the nonwoven fabrics require various characteristics in accordance with their applications, and improvements in their characteristics.

For example, excellent waterproofness and moisture permeability are required for nonwoven fabrics which are used for hygiene materials such as disposable diapers and sanitary napkins, and for backings for poultices, and the like. In addition, stretchability and bulkiness are also required depending on the sections to which the fabrics are used.

As a method of imparting stretchability to a nonwoven fabric, a method of using a thermoplastic elastomer as a raw material of a spunbonded nonwoven fabric (see, for example, Patent Literature 1), and a method of using a low crystalline polypropylene (see, for example, Patent Literature 2 and Patent Literature 3), and the like have been proposed.

Patent Literature 2 or Patent Literature 3 proposes that a highly crystalline polypropylene or a release agent is added to a low crystalline polypropylene in order to improve stickiness and the like of a spunbonded nonwoven fabric. Patent Literature 4 discloses discloses a multilayer of a nonwoven fabric containing a low crystalline polypropylene, a blended spunbonded nonwoven fabric of thermoplastic elastomer long fibers and thermoplastic resin long fibers

[Patent Literature 1] Japanese National-Phase Publication (JP-A) No. H07-503502
[Patent Literature 2] Japanese Patent Application Laid-Open (JP-A) No. 2009-62667
[Patent Literature 3] Japanese Patent Application Laid-Open (JP-A) No. 2009-79341
[Patent Literature 4] International Publication No. 2007/138733 Japanese Patent Applications 2018-201247 and 2017-197890 also disclose related products.

SUMMARY OF INVENTION

Technical Problem

According to the method described in Patent Literature 2 or Patent Literature 3, in order to prevent a spunbonded nonwoven fabrics containing a low crystalline polypropylene from adhering to various rotary units in apparatuses during steps such as embossing or to any other regions which are brought into contact with the nonwoven fabrics during production of the spunbonded nonwoven fabric using the low crystalline polypropylene, it is necessary to increase the amount of the highly crystalline polypropylene or release agent to be added to the low crystalline polypropylene. As a result, the resulting spunbonded nonwoven fabric tends to have a greater residual strain and to be inferior in stretchability. According to the method described in Patent Literature 4, stretchability is maintained by stacking a low crystalline polypropylene and an extensible spunbonded nonwoven fabric, but a further improvement in stretchability is strongly demanded.

In applications such as hygiene materials (such as disposable diapers and sanitary napkins), and backings for poultices, stress at extending is required to be small so that the materials can be mounted with a weak force, and stress at recovering is required to be large so as to prevent misalignment while being mounted. That is, in the above applications, it is required to increase the value of stretch characteristics (ratio of stress at recovering/stress at extending).

In applications such as hygiene materials (such as disposable diapers and sanitary napkins), and backings for poultices, it is required that the stress at recovery does not decrease in the range of room temperature (23°C) to body temperature (for example, the range of 23°C to 40°C), that is, a stress retention is excellent. As a result, even if hygiene materials such as disposable diapers and sanitary napkins rise to body temperature while being mounted, they will not easily shift.

In view of the above-described problems, it is an object of one aspect in the present invention to provide a multilayer nonwoven fabric, a stretchable multilayer nonwoven fabric, a fiber product, an absorbent article, and a sanitary mask which are excellent in stretch characteristics and are excellent in a stress retention.

Solution to Problem

Means for solving the above-mentioned problems are defined by the claims.

Advantageous Effects of Invention

One aspect in the present invention provides a multilayer nonwoven fabric, as defined by claim 1; a stretchable multilayer nonwoven fabric, as defined by claim 10; a fiber product as defined by claim 11; and an absorbent article, and a sanitary mask as respectively defined by claims 12 and 13; which are excellent in stretch characteristics and are excellent in a stress retention.

BRIEF DESCRIPTION OF DRAWINGS

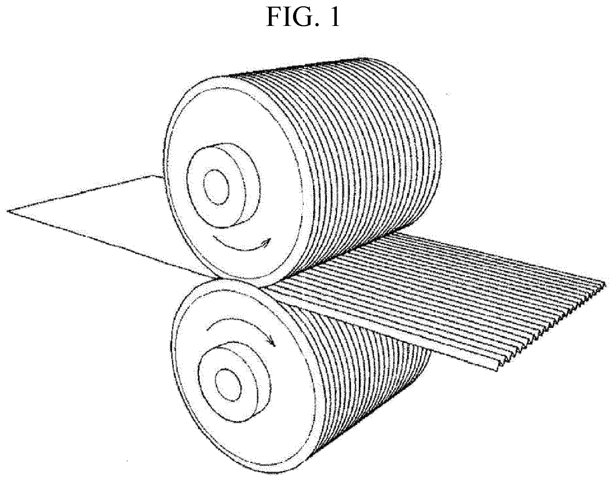

Fig. 1 is a schematic diagram of a gear stretching device.

DESCRIPTION OF EMBODIMENTS

Hereinafter, embodiments in the present disclosure will be described. These descriptions and examples illustrate the embodiments and do not limit the scope of the embodiments.

In the present disclosure, when numerical ranges are described in a stepwise manner, the values of the upper or lower limit of each numerical range may be substituted by the values of the upper or lower limit of the other numerical range. In the present disclosure, each numerical range may be substituted by the values described in the Examples.

In the present disclosure, each component may include more than one kind of substances corresponding to each component. When there are more than one kind of substances corresponding to each component in a composition, a content of the component refers to a total content of the substances, unless otherwise stated.

In the present disclosure, the "process" refers not only to a process that is independent from the other steps, but also to a step that cannot be clearly distinguished from the other steps, as long as the aim of the process is achieved. In the present disclosure, numerical ranges indicated using "to" mean a range including the numerical values described before and after "to" as the lower limit value and the upper limit value, respectively. When there are more than one kind of substances corresponding to each component in a composition, a content of the component refers to a total content of the substances, unless otherwise stated.

-Multilayer Nonwoven Fabric -

A multilayer nonwoven fabric in the present disclosure includes an elastic nonwoven fabric including an $\alpha$-olefin copolymer having a ratio of a storage elastic modulus E40 at 40°C and a storage elastic modulus E23 at 23°C (E40/E23) is 37% or more, and an extensible spunbonded nonwoven fabric disposed on at least one side of the elastic nonwoven fabric. The multilayer nonwoven fabric may be configured to include another layer.

The multilayer nonwoven fabric in the present disclosure includes an $\alpha$-olefin copolymer as an elastic nonwoven fabric. Therefore, compared with the case of using an elastic nonwoven fabric including no $\alpha$-olefin copolymer, for example, an elastic nonwoven fabric consisted of a polypropylene homopolymer, it is considered that the multilayer nonwoven fabric excellent in stretch characteristics and excellent in a stress retention can be obtained.

Further, the $\alpha$-olefin copolymer has a ratio of a storage elastic modulus E40 at 40°C and a storage elastic modulus E23 at 23°C (E40/E23) is 37% or more. Even in a temperature changing environment (for example, 40°C to 23°C), the decrease in elasticity of the elastic nonwoven fabric is easy to be suppressed. Therefore, it is considered that the multilayer nonwoven fabric excellent in a stress retention can be obtained.

In the multilayer nonwoven fabric in the present disclosure, the extensible spunbonded nonwoven fabric is disposed on at least one side of the elastic nonwoven fabric. Therefore, it is considered that the adherence of the multilayer nonwoven fabric to various rotating equipment and the like inside the device used for the emboss process and the like is easily prevented, and is excellent in moldability and productivity. Since the extensible spunbonded nonwoven fabric has extensibility, stretchability of the elastic nonwoven fabric due to excellent elasticity is easily maintained.

From the viewpoint of obtaining the multilayer nonwoven fabric which is excellent in stretch characteristics and is excellent in a stress retention, the extensible spunbonded nonwoven fabrics are preferably disposed on the both side of the elastic nonwoven fabric in the multilayer nonwoven fabric.

The multilayer nonwoven fabric in the present disclosure preferably has a basis weight of 360 $g/m^2$ or less, more preferably 240 $g/m^2$ or less, further preferably 150 $g/m^2$ or less, and particularly preferably from 120 $g/m^2$ to 15 $g/m^2$, even more preferably from 80 $g/m^2$ to 20 $g/m^2$, and extremely preferably from 70 $g/m^2$ to 25 $g/m^2$.

The basis weight ratio (compositional ratio) of the elastic nonwoven fabric and the extensible spunbonded nonwoven fabric may be appropriately determined according to various uses. For example, the basis weight ratio of the elastic nonwoven fabric and the extensible spunbonded nonwoven fabric (elastic nonwoven fabric:extensible spunbonded) is preferably in the range of 10:90 to 90: 10, more preferably in the range of 20:80 to 80:20, and further preferably in the range of 40:60 to 60:40.

When the basis weight ratio of the elastic nonwoven fabric is 10 or more, deterioration of stretchability of the multilayer nonwoven fabric tend to be suppressed. On the other hand, when the basis weight ratio of the elastic nonwoven fabric is 90 or less, the ratio in which the fibers constituting the elastic nonwoven fabric is beyond the extensible spunbonded nonwoven fabric layer to expose the surface, tend to be decreased. Therefore, the multilayer nonwoven fabric excellent in moldability and tactile feeling is easy to obtain.

In a case in which two or more elastic nonwoven fabrics (or two or more extensible spunbonded nonwoven fabrics) exist, the basis weight of the elastic nonwoven fabrics (or extensible spunbonded nonwoven fabrics) is the total of the basis weights of the two or more thereof.

The basis weight (g/m$^2$) of the multilayer nonwoven fabric is a value measured and determined as follows. Each basis weight of the elastic nonwoven fabric and extensible spunbonded nonwoven fabric is determined by the same manner.

Six test pieces of 200 mm (machine direction: MD) and 50 mm (lateral direction: CD) are sampled from the multilayer nonwoven fabric. Three sampling points are arbitrarily selected for both MD and CD (6 points in total). Subsequently, the mass (g) of each of the sampled test pieces is measured using a scale electric balance (manufactured by Kensei Co., Ltd.). The average value of the masses of the test pieces is determined. The determined average value is converted to the mass (g) per 1 m$^2$, and the first decimal place is rounded off to give a basis weight [g/m$^2$].

In the multilayer nonwoven fabric, a maximum load elongation in at least one direction is preferably 100% or more, more preferably 150% or more, and further preferably 220% or more.

The maximum load elongation (%) of the multilayer nonwoven fabric is a value measured and determined as follows.

Five test pieces of 200 mm (MD) and 50 mm (CD) are sampled from the multilayer nonwoven fabric. The test pieces are subjected to a tensile test using a constant speed extending type tensile tester under conditions of a chuck distance of 100 mm and a tensile speed of 100 mm/min, and the extension percentage of the test piece at the time when the load applied to the test piece becomes the maximum is measured. The average value of the five test pieces is determined and taken as a maximum load elongation.

[Elastic Nonwoven Fabric]

The elastic nonwoven fabric in the present disclosure includes an $\alpha$-olefin copolymer having a ratio of a storage elastic modulus E40 at 40°C and a storage elastic modulus E23 at 23°C (E40/E23) is 37% or more.

The basis weight of the elastic nonwoven fabric is preferably 120 g/cm$^2$ or less, more preferably 80 g/cm$^2$ or less, further preferably 50 g/cm$^2$ or less, particularly preferably from 40 g/cm$^2$ to 2 g/cm$^2$, even more preferably from 30 g/cm$^2$ to 5 g/cm$^2$, and extremely preferably from 25 g/cm$^2$ to 8 g/cm$^2$.

The fiber constituting the elastic nonwoven fabric preferably has a fiber diameter of 50 $\mu$m or less, more preferably 40 $\mu$m or less, and further preferably 30 $\mu$m or less. The fiber constituting the elastic nonwoven fabric may have a fiber diameter of 1.0 $\mu$m or more.

A method of producing the elastic nonwoven fabric is not particularly limited, and conventionally known methods can be applied. For example, the elastic nonwoven fabric may be the elastic nonwoven fabric produced by the method such as a spunbond method, a melt blow method, and a flash spinning method. Among the above, from the viewpoint of making the fibers forming the nonwoven fabric long fibers, an elastic spunbonded nonwoven fabric produced by the spunbond method is preferable.

($\alpha$-Olefin Copolymer)

The elastic nonwoven fabric includes an $\alpha$-olefin copolymer.

The $\alpha$-olefin copolymer represents a copolymer in which two or more kinds of copolymerization components having an $\alpha$-olefin skeleton are copolymerized.

Examples of copolymerization components having an $\alpha$-olefin skeleton include $\alpha$-olefins such as ethylene, propylene, 1-butene, 1-pentene, 3-methyl-1-pentene, 4-methyl-1-pentene, 1-hexene, 1-octene, 1-decene, 1-dodecene, 1-tetradecene, 1-hexadecene, 1-octadecene, and 1-eikosen.

Among the above, from the viewpoint of making the multilayer nonwoven fabric lower stress and more excellent in stretchability, the $\alpha$-olefin copolymer preferably includes an ethylene and propylene copolymer in which ethylene (written as "C2" in Tables 1 and 2 below) and propylene (written as "C3" in Tables 1 and 2 below) are copolymerization components.

The content ratio of a structural unit derived from ethylene (hereinafter, it is also simply referred to as "ethylene content") in the ethylene and propylene copolymer is preferably from 1% by mass to 50% by mass, more preferably from 5% by mass to 25% by mass, further preferably from 10% by mass to 20% by mass, and particularly preferably from 12% by mass to 18% by mass.

The α-olefin copolymer may be any of an alternating copolymer, a graft copolymer, a block copolymer and a random copolymer.

The ratio of a storage elastic modulus E40 at 40°C and a storage elastic modulus E23 at 23°C (E40/E23) of an α-olefin copolymer is, from the viewpoint of obtaining the multilayer nonwoven fabric excellent in a stress retention, 37% or more. The ratio (E40/E23) is preferably the higher value, more preferably 40% or more, further preferably 45% or more, and particularly 50% or more. The upper limit of the ratio (E40/E23) is not particularly limited, and may be 100% or less, may be 95% or less, or may be 90% or less.

Examples of a method of setting the ratio E40/E23 of a storage elastic modulus to the above specific range include a method to use a copolymer of ethylene and propylene as an a copolymer of ethylene and propylene.

From the viewpoint of making the multilayer nonwoven fabric more excellent in stretchability, a storage elastic modulus E23 at 23°C of an α-olefin copolymer is preferably 30 MPa or less, more preferably 22 MPa or less, further preferably 20 MPa or less, and particularly preferably 18 MPa or less.

From the viewpoint of making the multilayer nonwoven fabric lower stress and more excellent in stretchability, a storage elastic modulus E40 at 40°C of an α-olefin copolymer is preferably 10 MPa or less, and more preferably 9 MPa or less.

The storage elastic modulus E23 at 23°C of an α-olefin copolymer may be 5 MPa or more, or may be 10 MPa or more.

The storage elastic modulus E40 at 40°C of an α-olefin copolymer may be 3 MPa or more, or may be 5 MPa or more.

Each storage elastic modulus of an α-olefin copolymer is a value measured by the following equipment and conditions.

Temperature: 23°C or 40°C
Equipment: RSA-III (manufactured by TA Instruments)
Deformation mode: Pulling mode
Temperature range: -20°C to 120°C
Temperature increasing rate: 2°C/min
Deformation frequency: 10 Hz
Initial distortion: 0.1%
Measurement temperature interval: 0.3°C
Environment: under nitrogen atmosphere

A density (ASTM D 1505) of an α-olefin copolymer is preferably in a range of from 0.850 $g/cm^3$ to 0.950 $g/cm^3$, more preferably in a range of from 0.855 $g/cm^3$ to 0.900 $g/cm^3$, and further preferably in a range of from 0.860 $g/cm^3$ to 0.895 $g/cm^3$.

The density of an α-olefin copolymer is a value obtained by measuring according to the density gradient method of JIS K7112 (1999).

From the viewpoint of making the multilayer nonwoven fabric more excellent in stretchability, a tensile modulus of an α-olefin copolymer is preferably 30 MPa or less, more preferably 20 MPa or less, and further preferably 15 MPa or less. The upper limit of the tensile modulus of an α-olefin copolymer is not particularly limited, and for example, may be 5 MPa or less.

The tensile modulus of an α-olefin copolymer is a value obtained by measuring according to the density gradient method of JIS K7161 (2011).

A molecular weight distribution (Mw/Mn) of an α-olefin copolymer is preferably from 1.5 to 5.0. From the view point of obtaining fibers having good spinnability and excellent fiber strength, Mw/Mn is more preferably from 1.5 to 4.5.

A weight average molecular weight (Mw) and a number average molecular weight (Mn) are values determined by GPC (gel permeation chromatography), and values measured by the following conditions. The weight average molecular weight (Mw) is the weight average molecular weight in terms of polystyrene, and the molecular weight distribution (Mw/Mn) is a value calculated from the number average molecular weight (Mn) and the weight average molecular weight (Mw) measured in the same manner.

<GPC measurement conditions>

Column: TOSO GMHHR-H (S) HT
Detector: RI detector for liquid chromatogram WATERS 150C
Solvent: 1,2,4-trichlorobenzene
Measurement temperature: 145°C
Flow velocity: 1.0 ml/min
Sample concentration: 2.2 mg/ml
Injection amount: 160 μl
Calibration curve: Universal Calibration
Analysis program: HT-GPC (Ver.1.0)

A melt flow rate of an α-olefin copolymer is not particularly limited, and for example, preferably from 1 g/10 min to 100 g/10 min, more preferably from 10 g/10 min to 80 g/10 min, further preferably from 15 g/10 min to 70 g/10 min, and particularly preferably from 15 g/10 min to 50 g/10 min.

The melt flow rate of an α-olefin copolymer is measured under the condition of ASTM D1238, 230°C, and load: 2.16 kg.

An α-olefin copolymer may be a synthetic product or a commercially available product. When the α-olefin copolymer is a synthetic product, the α-olefin copolymer can be prepared by polymerizing or copolymerizing monomer by a conventionally known polymerization method such as a gas phase method, a bulk method, a slurry method, or a solution method, in the presence of a conventionally known catalyst such as a Ziegler-Natta catalyst or a metallocene catalyst.

Examples of commercially available products of α-olefin copolymers include TAFMER (manufactured by Mitsui Chemicals) and Vistamaxx series (manufactured by ExxonMobil Chemical Company).

A composition of an α-olefin copolymer can be analyzed by using a conventionally known methods (for example, IR analysis, NMR analysis, and microanalysis).

A ratio of an α-olefin copolymer with respect to the total amount of the elastic nonwoven fabric is from 90% by mass to 100% by mass, and preferably from 98% by mass to 100% by mass.

In a case in which an α-olefin copolymer includes an ethylene and propylene copolymer, from the viewpoint of stretch characteristics in the multilayer nonwoven fabric, a ratio of the ethylene and propylene copolymer with respect to the total amount of the elastic nonwoven fabric is 90% by mass to 100% by mass.

In a case in which an α-olefin copolymer is an ethylene and propylene copolymer, from the viewpoint of stretch characteristics in the multilayer nonwoven fabric, a degree of crystallinity of the α-olefin copolymer is preferably from 1% to 15%, more preferably from 1% to 13%, further preferably from 2% to 10%, and particularly preferably from 4% to 10%.

The degree of crystallinity of the α-olefin copolymer is calculated from the melting heat curve derived from melting of a principal component in melting endothermic curve obtained by being held at -100°C for 5 minutes under a nitrogen atmosphere and thereafter heated at a temperature increasing rate of 10°C/min, using a differential scanning calorimeter (DSC). Specifically, the degree of crystallinity can be calculated by using a differential scanning calorimeter (DSC-7 manufactured by Perkin Elmer) and using the following formula and the melting heat curve derived from melting of a principal component in melting endothermic curve obtained by holding 5 mg of a sample at -100°C for 5 minutes under a nitrogen atmosphere and thereafter heating the sample at a temperature increasing rate of 10°C/min.

$$\text{Degree of Crystallinity} = (\Delta H/\Delta H0) \times 100 \ (\%)$$

In the formula, $\Delta H$ is a heat of fusion (J/g) obtained from the melting heat curve derived from the melting of a main component of an α-olefin copolymer including ethylene and propylene, and $\Delta H0$ is a heat of fusion (J / g) of the perfect crystal of the main component. Is. That is, when the main component is ethylene, $\Delta H0$ is 293 J/g, and when the main component is propylene, $\Delta H0$ is 210 J/g.

In a case in which an α-olefin copolymer is an ethylene and propylene copolymer, a melting point of the ethylene and propylene copolymer may be 130 °C or less, may be 115°C or less, may be 100°C or less, may be from 40°C to 85°C, or may be from 40°C to 60°C.

The melting point of an α-olefin copolymer is defined as the peak top of the peak observed on the lowest side of a melting endothermic curve obtained by being held at -100°C for 5 minutes under a nitrogen atmosphere and thereafter heated at a temperature increasing rate of 10 °C/min, using a differential scanning calorimeter (DSC). Specifically, the melting point can be determined by using a differential scanning calorimeter (DSC-7, manufactured by Perkin Elmer), holding 5 mg of a sample at -100°C under a nitrogen atmosphere for 5 minutes, thereafter heating the sample at a temperature increasing rate of 10°C/min to obtain a melting endothermic curve, and determining the temperature as the peak top of the peak observed on the lowest temperature side of the curve.

[Extensible Spunbonded Nonwoven Fabric]

In the extensible spunbonded nonwoven fabric in the present disclosure, a maximum load elongation in at least one direction is 45% or more, preferably 70% or more, further preferably 100% or more, and particularly preferably 150% or more. It is preferable that the extensible spunbonded nonwoven fabric is a nonwoven fabric having a property with almost no elastic recovery. The maximum load elongation (%) of the extensible spunbonded nonwoven fabric is determined by the same manner as the maximum load elongation of the multilayer nonwoven fabric. In the extensible spunbonded nonwoven fabric in the present disclosure, the maximum load elongation in at least one direction may be 600% or less, or may be 500% or less.

The extensible spunbonded nonwoven fabric preferably has a basis weight of 120 $g/m^2$ or less, more preferably 80 $g/m^2$ or less, further preferably 50 $g/m^2$ or less, and particularly preferably from 40 $g/m^2$ to 5 $g/m^2$, even more preferably from 30 $g/m^2$ to 5 $g/m^2$, and extremely preferably from 25 $g/m^2$ to 8 $g/m^2$.

The fiber constituting the extensible spunbonded nonwoven fabric preferably has a fiber diameter of 50 $\mu$m or less, more preferably 40 $\mu$m or less, and further preferably 30 $\mu$m or less. The fiber constituting the extensible spunbonded nonwoven fabric may have a fiber diameter of 1.0 $\mu$m or more.

The extensible spunbonded nonwoven fabric may be any of a concentric sheath-core composite fiber having a core portion and a sheath portion, a sea-island type composite fiber having a sea portion and an island portion, a parallel type composite fiber, and a crimped composite fiber. The extensible spunbonded nonwoven fabric is preferably a concentric sheath-core composite fiber or a sea-island type composite fiber.

In the extensible spunbonded nonwoven fabric including the concentric sheath-core composite fiber, it is preferable that the core portion is a low MFR olefin polymer having MFR in the range of from 1 g/10 min to 1000 g/10 min, the sheath portion is a high MFR olefin polymer having MFR in the range of from 1 g/10 min to 1000 g/10 min, and the difference in MFR between the low MFR olefin polymer and the high MFR olefin polymer is 1 g/10 min or more. The difference in MFR is preferably 15 g/10 min or more, more preferably 30 g/10 min or more, and particularly preferably 40 g/10 min or more. The difference in MFR may be 100 g/10 min or less, or may be 70 g/10 min or less.

The extensible spunbonded nonwoven fabric including the sea-island type composite fiber may be a sea-island type composite in which the sea portion is a propylene polymer (preferably a propylene homopolymer) and the island portion is an ethylene polymer (preferably a high density polyethylene).

Examples of the extensible spunbonded nonwoven fabric include a nonwoven fabric using one kind or two or more kinds of olefin polymer compositions described later.

(Olefin Polymer Composition)

The extensible spunbonded nonwoven fabric includes an olefin polymer, or is formed from an olefin polymer composition including an olefin polymer. An olefin polymer may be a polyolefin elastomer.

The olefin polymer composition may include, as an optional component, various known additives such as an antioxidant, a heat-resistant stabilizer, a weather-resistant stabilizer, an antistatic agents, a slip agents, an antifogging agents, a lubricant, a dye, a pigment, a natural oil, a synthetic oil, a wax, and a hydrophilic agent as long as the object of the present disclosure is not impaired.

The olefin polymer is preferably a polymer having crystallinity. Examples of the crystalline component in the polymer having crystallinity include poly1-butene and poly4-methyl-1-pentene. The olefin polymer may be used alone or in combination of two or more.

Examples of the olefin polymer include a homopolymer or a copolymer of $\alpha$-olefins such as ethylene, propylene, 1-butene, 1-hexene, 4-methyl-1-pentene and 1-octene. Examples of the copolymer of $\alpha$-olefins include an ethylene polymer and a propylene polymer.

Examples of the ethylene polymer include ethylene homopolymers such as a high pressure low density polyethylene, a linear low density polyethylene (so-called LLDPE), a high density polyethylene (so-called HDPE), and a random copolymer or a block copolymer of ethylene and an $\alpha$-olefin.

A density of the ethylene polymer is not particularly limited, and for example, is preferably from 0.94 $g/cm^3$ to 0.97 $g/cm^3$, more preferably from 0.95 $g/cm^3$ to 0.97 $g/cm^3$, and further preferably from 0.96 $g/cm^3$ to 0.97 $g/cm^3$.

MFR of the ethylene polymer is not particularly limited as long as the ethylene polymer has spinnability, and for example, from the viewpoint of causing extensibility, is preferably from 0.1 g/10 min to 100 g/10 min, more preferably from 0.5 g/10 min to 50 g/10 min, and further preferably from 1 g/10 min to 30 g/10 min.

The propylene polymer generally refers to a crystalline resin produced and sold under the name of polypropylene. The propylene polymer is preferably a propylene homopolymer propylene or a copolymer in which propylene is a main component.

Examples of the copolymer in which propylene is a main component include copolymers in which $\alpha$-olefins having 2 or more carbon atoms (preferably $\alpha$-olefins having 2 to 8 carbon atoms) such as ethylene, 1-butene, 1-pentene, 1-hexene, 1-octene, and 4-methyl-1-pentene are copolymerization components (however, alkene having 3 carbon atoms, that is, propylene is excluded). The copolymer in which propylene is a main component may be any of a random copolymer, a block copolymer and the like.

A melting point (Tm) of the propylene homopolymer is preferably 155°C or more, and more preferably from 157°C to 165°C.

A melting point (Tm) of the copolymer in which propylene is a main component is 130°C or more and less than 155°C, and more preferably from 130°C to 150°C.

MFR of the propylene polymer is not particularly limited as long as the propylene polymer can be melt-spun. The MFR of the propylene polymer is preferably from 1 g/10 min to 1000 g/10 min, more preferably 5 g/10 min to 500 g/10 min, and further preferably 10 g/10 min to 100 g/10 min.

The extensible spunbonded nonwoven fabric may include a polymer other than the olefin polymer (hereinafter, it is also referred to as "another polymer") or may not include another polymer. Examples of another polymer include thermoplastic elastomers and thermoplastic resins other than olefin polymers.

Specific examples of thermoplastic elastomers includes a styrene elastomer, a polyester elastomer, a polyamide elastomer, a thermoplastic polyurethane elastomer, a vinyl chloride elastomer, and a fluorine elastomer.

Specific examples of thermoplastic resins other than olefin polymers include polyester (polyethylene terephthalate, polybutylene terephthalate, polyethylene naphthalate, etc.), polyamide (nylon-6, nylon-66, polymethoxylen adipamide, etc.), polyvinyl chloride, polyimide, ethylene-vinyl acetate copolymer, ethylene-vinyl acetate-vinyl alcohol copolymer, ethylene-(meth) acrylic acid copolymer, ethylene-acrylic acid ester-carbon monoxide copolymer, polyacrylonitrile, polycarbonate, and polystyrene.

A content of the olefin polymer in the extensible spunbonded nonwoven fabric is more than 90% by mass, preferably from 95% by mass to 100% by mass with respect to the total of the olefin polymer and another polymer (thermoplastic elastomers and thermoplastic resins other than olefin polymers).

The extensible spunbonded nonwoven fabric does not include another polymer or a content of another polymer (thermoplastic elastomers and thermoplastic resins other than olefin polymers) in the extensible spunbonded nonwoven fabric is more than 0% by mass and less than 10% by mass, and preferably more than 0% by mass and 5% by mass or less with respect to the total of the olefin polymer and another polymer.

In a case in which the olefin polymer composition includes the propylene polymer and the ethylene polymer, a content of the propylene polymer is preferably from 80% by mass to 99% by mass, and more preferably from 84% by mass to 96% by mass with respect to the total amount of the olefin polymer composition. On the other hand, a content of the ethylene polymer is preferably from 20% by mass to 1% by mass, and more preferably from 16% by mass to 4% by mass with respect to the total amount of the olefin polymer composition (with the proviso that propylene-based polymer + ethylene-based polymer = 100% by mass).

(Specific Examples of Extensible Spunbonded Nonwoven Fabric)

Examples of the extensible spunbonded nonwoven fabric include an extensible spunbonded nonwoven fabric satisfying the following requirements (1) to (3).

(1) A spunbonded nonwoven fabric composed of two or more kinds of olefin polymers in which the difference in crystallization induction time in flow-induced phase separation is 100 seconds or more, and using a sheath-core composite fiber, a parallel type composite fiber (side-by-side type composite fiber) or a crimped composite fiber.

Examples of the above two or more kinds of olefin polymers may include a high melting point propylene polymer and a low melting point propylene polymer.

(2) A spunbonded nonwoven fabric composed of an olefin polymer composition including a propylene polymer and an ethylene polymer, and using a sheath-core composite fiber, a sea-island type composite fiber, a parallel type composite fiber or a crimped composite fiber. Particularly, as the olefin polymer composition, those shown below are preferable.

(2-1) An olefin polymer composition composed of from 80% by mass to 99% by mass of a propylene polymer and from 20% by mass to 1% by mass of an ethylene polymer.
(2-2) An olefin polymer composition including a high melting point propylene polymer in which MFRs are the same or different, and the melting point of the propylene polymer is in the range of from 157°C to 165°C.

For example, the above propylene polymer may be a propylene polymer obtained by copolymerizing a propylene homopolymer, and a low melting point random copolymer of a propylene and α-olefin, having a melting point in the range of from 130°C to 150°C.

(3) A spunbonded nonwoven fabric using a concentric sheath-core composite fiber in which a core portion is a low MFR propylene polymer having MFR in a range of from 1 g/10 min to 200 g/10 min, a sheath portion is a high MFR propylene polymer having MFR in a range of from 16 g/10 min to 215 g/10 min, and a difference between MFR of the core portion and MFR of the sheath portion is 15 g/10 min or more.

Examples of the extensible spunbonded nonwoven fabrics satisfying the above requirement (1) to (3), include the extensible spunbonded nonwoven fabrics of the following (A) and (B).

(A) A spunbonded nonwoven fabric using a concentric sheath-core composite fiber composed of a sheath-core composite fiber, in which a core portion is a low MFR and high melting point propylene polymer (preferably, propylene homopolymer) having MFR in a range of from 1 g/10 min to 200 g/10 min and a melting point in a range of 157°C to 165°C, a sheath portion is a high MFR and low melting point propylene and $\alpha$-olefin random copolymer having MFR in a range of from 1 g/10 min to 200 g/10 min and a melting point in a range of 130°C to 150°C, and a difference between MFR of the core portion and MFR of the sheath portion is 1 g/10 min or more, a parallel type composite fiber or a crimped composite fiber

(B) A spunbonded nonwoven fabric composed of a concentric sheath-core composite fiber in which a core portion is a low MFR propylene polymer (preferably, propylene homopolymer) having MFR in a range of from 1 g/10 min to 200 g/10 min, a sheath portion is a high MFR propylene polymer (preferably, propylene homopolymer) having MFR in a range of from 31 g/10 min to 230 g/10 min, and a difference between MFR of the core portion and MFR of the sheath portion is 30 g/10 min or more.

In the above (B), the core portion may be a low MFR propylene polymer having MFR in a range of from 10 g/10 min to 50 g/10 min, and the sheath portion may be a high MFR propylene polymer having MFR in a range of from 50 g/10 min to 100 g/10 min. A difference between MFR of the core portion and MFR of the sheath portion may be from 30 g/10 min to 100 g/10 min, or may be from 40 g/10 min to 80 g/10 min

(Another Layer)

In the multilayer nonwoven fabric in the present disclosure, another layer may be stacked depending on various uses. Another layer stacked on the multilayer nonwoven fabric in the present disclosure is not particularly limited, and various layers may be stacked depending on uses.

Specific examples of another layer include a knitted fabric, a woven fabric, a nonwoven fabric other than the elastic nonwoven fabric or the extensible spunbonded nonwoven fabric, and a film. A method of further stacking (adhering) another layer on the multilayer nonwoven fabric in the present disclosure is not particularly limited, and various methods such as a thermal fusion method such as thermal embossing or ultrasonic fusion; a mechanical confounding method such as needle punch or water jet; a method of using an adhesive such as a hot melt adhesive or a urethane adhesive; and extrusion laminating may be employed.

In a case in which the multilayer nonwoven fabric in the present disclosure includes a nonwoven fabric other than the elastic nonwoven fabric or the extensible spunbonded nonwoven fabric, Examples of the nonwoven fabric include various known nonwoven fabrics such as a spunbonded nonwoven fabric, a meltblown nonwoven fabric, a wet nonwoven fabric, a dry nonwoven fabric, a dry pulp nonwoven fabric, a flash spun nonwoven fabric, and a split non-woven fabric. The nonwoven fabric may be a stretchable nonwoven fabric or a nonstretchable nonwoven fabric. Herein, the nonstretchable nonwoven fabric refers to a nonwoven fabric which does not cause return stress after extending it in MD (machine direction of the nonwoven fabric, longitudinal direction) or CD (direction perpendicular to the machine direction of the nonwoven fabric, lateral direction).

In a case in which the multilayer nonwoven fabric in the present disclosure includes a film, the film is preferably an air breathable (moisture permeable) film from the viewpoint of maintaining air breathability and hydrophilicity which are features of the multilayer nonwoven fabric in the present disclosure. Examples of the air breathable film include various known air breathable films such as a film made of moisture permeable thermoplastic elastomer (such as a polyurethane elastomer, a polyester elastomer, or a polyamide elastomer), and a porous film obtained by stretching a film made of a thermoplastic resin containing inorganic fine particles or organic fine particles to create pores in the film. As the thermoplastic resin used for the porous film, polyolefins such as a high pressure low density polyethylene, a linear low density polyethylene (so-called LLDPE), a high density polyethylene, polypropylene, a polypropylene random copolymer, and combinations thereof are preferable. In a case in which it is not necessary to maintain air breathability and hydrophilicity of the multilayer nonwoven fabric, thermoplastic resin films made of polyethylene, polypropylene, or combinations thereof and the like may be used.

(Producing Method of Multilayer nonwoven fabric)

The multilayer nonwoven fabric in the present disclosure can be produced by a known nonwoven fabric producing method using the elastic nonwoven fabric including an α-olefin copolymer, the extensible spunbonded nonwoven fabric and an additive used if necessary.

As an example of a method of producing the multilayer nonwoven fabric, a method of using a nonwoven fabric producing apparatus having at least two-line spinning devices will be described below. The example described below is an example of a method of producing the multilayer nonwoven fabric using an olefin polymer as the extensible spunbonded nonwoven fabric and an α-olefin copolymer as the elastic nonwoven fabric.

In the multilayer nonwoven fabric in the present disclosure, from the viewpoint of the production, an aspect in which the extensible spunbonded nonwoven fabric is disposed on the plane at the side which is in contact with the rotating equipment accompanying the nonwoven fabric producing apparatus is preferable.

First, an olefin polymer, or two or more kinds of olefin polymers if necessary are melted by an extruder provided in a first-line spinning device or two or more extruders, introduced to a spinneret (die) fitted with a large number of spinning holes (nozzles) or sheath-core spinning holes if necessary, and discharged. Thereafter, the long fibers including the melt-spun olefin polymer are introduced into a cooling chamber, and cooled by cooling air. Then, the long fibers are then stretched (towed) by stretching air, and the extensible spunbonded nonwoven fabric is deposited on a moving collection surface.

On the other hand, the resin composition including an α-olefin copolymer in the present disclosure is melted by an extruder provided in a second-line spinning device, introduced to a spinneret (die) fitted with a large number of spinning holes (nozzles), and discharged. Thereafter, the long fibers including the melt-spun resin composition are introduced into a cooling chamber, and cooled by cooling air. Then, the long fibers are stretched (towed) by stretching air, and deposited on the extensible spunbonded nonwoven fabric to form an elastic nonwoven fabric.

If necessary, the extensible spunbonded nonwoven fabric may be deposited on the elastic nonwoven fabric by using a third-line spinning device.

The melting temperatures of polymers including the elastic nonwoven fabric or the extensible spunbonded nonwoven fabric are not particularly limited as long as the temperatures are equal to or higher than the softening temperature or melting temperature of each polymer and less than the thermal decomposition temperature. The temperature of the spinneret depends on the type of the polymer to be used. For example, in a case in which an ethylene and propylene copolymer as an α-olefin copolymer is used, the temperature of the spinneret is preferably from 180°C to 240°C, more preferably from 190 to 230°C, and further preferably from 200 to 225°C.

The temperature of the cooling air is not particularly limited as long as the polymer can be solidified, and is preferably from 5°C to 50°C, more preferably from 10°C to 40°C, and further preferably from 15°C to 30°C. A stretching air velocity is preferably from 100 m/min to 10,000 m/min, and more preferably from 500 m/min to 10,000 m/min.

The multilayer nonwoven fabric in the present disclosure preferably has a structure in which at least a part of the elastic nonwoven fabric and at least a part of the extensible spunbonded nonwoven fabric are thermally fusion bonded. At this time, at least a part of the elastic nonwoven fabric and at least a part of the extensible spunbonded nonwoven fabric may be compacted with nip rolls before thermal fusion bonding.

The method of thermal fusion bonding is not particularly limited, and it can be selected from various known methods. Examples of prebonding methods include a method of using means such as ultrasonic waves, thermal embossing using embossing rolls, and a method using hot air through. Among these, from the viewpoint that the long fibers are efficiently stretched during streching, thermal embossing is preferable, and the temperature range thereof is preferably from 40°C to 115°C.

In a case in which a part of the multilayer is thermally fusion bonded by thermal embossing, the emboss area ratio is preferably from 5% to 30%, and more preferably from 5% to 20%. The non-embossed unit area is preferably from 0.5 mm$^2$ or more, and more preferably from 4 mm$^2$ to 40 mm$^2$. The non-embossed unit area is the maximum area of a quadrangle inscribed in emboss in a non-embossed portion of minimum units surrounded by embossed portions in all directions. Examples of the shape of the mark include a circle, an ellipse, an oval, a square, a rhombus, a rectangle, a square, and continuous shapes based on these shapes.

<Stretchable Multilayer Nonwoven Fabric>

The stretchable multilayer nonwoven fabric in the present disclosure is a multilayer nonwoven fabric having stretchability obtained by stretching the multilayer nonwoven fabric.

The stretchable multilayer nonwoven fabric in the present disclosure is a stretching product of the multilayer nonwoven fabric obtained by stretching the multilayer nonwoven fabric can be obtained by stretching the multilayer nonwoven fabric. The stretching method is not particularly limited, and conventionally known methods can be applied. The stretching method may be such that the multilayer nonwoven fabric is stretched partially or entirely. The stretching method may be such that the multilayer nonwoven fabric is stretched uniaxially or biaxially. Examples of the method of stretching the multilayer nonwoven fabric in a machine direction (MD) include a method of passing the partially fusion bonded mixed fibers through two or more pairs of nip rolls. At this time, the partially fusion bonded multilayer nonwoven fabric can be stretched by increasing the rotational speeds of the nip rolls in order in the machine direction. Gear stretching can also be performed using a gear stretching device shown in Fig. 1.

The lower limit of the stretching ratio is preferably 50% or more, more preferably 100% or more, and further preferably 200% or more. On the other hand, the upper limit of the stretching ratio is preferably 1000% or less, and more preferably 500% or less.

In the case of uniaxial stretching, the machine direction (MD) or a direction perpendicular thereto (CD) preferably satisfies the above stretching ratio. In the case of biaxial stretching, at least one of the machine direction (MD) or a direction perpendicular thereto (CD) preferably satisfies the above stretching ratio.

By stretching at the above stretching ratio, all the (long) fibers forming the elastic nonwoven fabric and the extensible spunbonded nonwoven fabric are stretched. The long fibers forming the extensible spunbonded nonwoven fabric layer are plastically deformed to be extended (lengthened) in accordance with the above stretching ratio.

In a case in which the stress is released after the multilayer nonwoven fabric is stretched, the (long) fibers forming the elastic nonwoven fabric are elastically recovered, and the long fibers forming the extensible spunbonded nonwoven fabric are folded without being elastically recovered. As a result, the multilayer nonwoven fabric exhibits bulkiness. In addition, since the long fibers forming the extensible spunbonded nonwoven fabric become thinner, the long fibers can improve flexibility and tactile feeling, and can give an extension stop function.

<Fiber Product>

The fiber product in the present disclosure includes the multilayer nonwoven fabric or the stretchable multilayer nonwoven fabric in the present disclosure. The fiber product is not particularly limited, and examples thereof include absorbent articles such as disposable diapers and sanitary articles, hygiene articles such as sanitary masks, medical articles such as bandages, clothing materials, and packaging materials. The fiber product in the present disclosure preferably includes the multilayer nonwoven fabric or the stretchable multilayer nonwoven fabric in the present disclosure as an elastic member.

EXAMPLES

Hereinafter, the invention will be more specifically described with reference to Examples, but the invention is not limited to these Examples. Unless otherwise specified, "part" means "part by mass".

-Preparation of Materials-

As raw materials of the elastic nonwoven fabric, the following materials were prepared · α-olefin copolymer 1A (propylene/ethylene copolymer)

product name "Vistamaxx™6202", manufactured by Exxon Mobil Corporation, MFR (230°C., load 2.16 kg): 20 g/10 min, ethylene content: 15% by mass, tensile modulus: 9.8 MPa. •α-olefin copolymer 1B (propylene/ethylene copolymer)

product name "Vistamaxx™7050FL", manufactured by Exxon Mobil Corporation, MFR (230°C, load 2.16 kg): 48 g/10 min, ethylene content: 13% by mass, tensile modulus: 14.4 MPa.

•The synthesis of α-olefin copolymer 1C (low crystalline polypropylene)

To a stainless steel reactor having an inner volume of 0.2 $m^3$ equipped with a stirrer, n-heptane at 20 L/h, triisobutylaluminum at 15 mmol/h, and a catalyst component obtained by preliminarily contacting dimethylanilinium tetrakispentafluorophenylborate, (1,2'-dimethylsilylene)(2,1'-dimethylsilylene)-bis(3-trimethylsilylmethylindenyl)zirconium dichloride, triisobutylaluminum, and propylene at 6 μmol/h per zirconium were continuously supplied. Propylene and hydrogen were continuously supplied in a state where a hydrogen concentration in a gas phase portion was 8 mol% at a polymerization temperature of 70°C and the total pressure in the reactor was maintained at 0.7 MPa·G. To the obtained polymerization solution, 1000 ppm of SUMILIZER GP (manufactured by Sumitomo Chemical Co., Ltd.) was added, and the solvent was removed to obtain a propylene polymer.

The propylene polymer thus obtained had a weight-average molecular weight (Mw) of $1.2 \times 10^4$ and Mw/Mn of 2. According to NMR measurement, [mmmm] was 46 mol%; [rrrr]/(1-[mmmm]) was 0.038; [rmrm] was 2.7 mol%; [mm] $\times$ [rr]/[mr]$^2$ was 1.5; and a tensile modulus was 32.9 MPa.

Herein, [mmmm] is a mesopentad fraction, [rrrr] is a racemic pentad fraction, [rmrm] is a racemic meso racemic mesopentad fraction and [mm], [rr] and [mr] are triad fractions. These values can be determined by the method described in International Publication No. WO 2016/143834.

[Example 1]

A propylene homopolymer (hereinafter "polymer 2A"; "PP" in Tables 1 and 2) having MFR (measured at 230°C under a load of 2.16 kg in accordance with ASTM D 1238) of 8.5 g/10 min, a density 0.91 g/cm$^3$, and a melting point of 160°C was melted using an extruder having a diameter of 50 mm. Independently, a propylene homopolymer (hereinafter "polymer 2B"; "PP" in Tables 1 and 2) having MFR (measured in accordance with ASTM D 1238 at 230°C under a load of 2.16 kg) of 60 g/10 min, a density of 0.91 g/cm$^3$, and a melting point of 160°C was melted using an extruder having a diameter of 75 mm. Thereafter, composite melt spinning was performed according to a spunbonding method using a spunbonded nonwoven fabric molding machine (length in a direction perpendicular to a machine direction on a collection surface: 800 mm) having a spinneret (die, number of holes: 2887) capable of molding a concentric sheath-core composite fiber in which the "polymer 2A" was a core and the "polymer 2B" was a sheath in such conditions that both a resin temperature and a die temperature were 250°C; a cooling air temperature was 20°C; and a stretching air velocity was 3750 m/min to deposit an extensible spunbonded nonwoven fabric composed of the concentric sheath-core composite fiber having a core portion and a sheath portion at a mass ratio of 10/90 on the collection surface.

Subsequently, α-olefin copolymer 1A was melted using a single-screw extruder with a screw diameter of 75 mmφ, and thereafter, melt spinning was performed according to a spunbonding method using a spunbonded nonwoven fabric molding machine (length in a direction perpendicular to a machine direction on a collection surface: 800 mm) having a spinneret (die, number of holes: 808) in such conditions that both a resin temperature and a die temperature were 215°C; a cooling air temperature was 20°C; and a stretching air velocity was 3750 m/min to deposit the elastic nonwoven fabric (elastic spunbonded non-woven fabric) as the second layer on the depositional surface. In this process, spinnability of α-olefin copolymer 1A was very good.

Subsequently, as the third layer, sheath-core composite fibers similar to those in the first layer were deposited by the same method to form a three-layer deposit. This deposit was heat-pressurized with an embossing roll (embossed area ratio 18%, embossed temperature 70°C), and the multilayer nonwoven fabric in which the total basis weight was 30.0 g/m$^2$, and the basis weight of the first layer and third layer were 10.0 g/m$^2$, and the basis weight of the elastic nonwoven fabric layer as the second layer was 10.0 g/m$^2$ (the mass fraction of the elastic nonwoven fabric layer to the whole is 33.3%).

The multilayer nonwoven fabric thus obtained had almost no adhesion to the surface of the metal roll in the embossing process, and moldability was good. In addition, when the multilayer fabric was wound into a roll state, roll blocking (a phenomenon in which the overlapping nonwoven fabrics adhere to each other and the roll solidifies) did not occur, and the fabric could be easily pulled out.

[Example 2]

The multilayer nonwoven fabric was obtained in the same manner as Example 1 except that the raw material of the elastic nonwoven fabric was changed from α-olefin copolymer 1A to α-olefin copolymer 1B.

[Comparative Example 1]

The multilayer nonwoven fabric was obtained in the same manner as Example 1 except that the raw material of the elastic nonwoven fabric was changed from α-olefin copolymer 1A to α-olefin homopolymer 1C.

[Example 3]

The multilayer nonwoven fabric was obtained in the same manner as Example 1 except that the basis weight of each extensible spunbonded nonwoven fabric in the first layer and the third layer and the basis weight of the elastic nonwoven fabric in the second layer were change from 10.0 g/cm$^2$ to 16.7 g/cm$^2$.

[Example 4]

The multilayer nonwoven fabric was obtained in the same manner as Example 1 except that the basis weight of each extensible spunbonded nonwoven fabric in the first layer and the third layer was changed from 10.0 g/cm$^2$ to 15.6 g/cm$^2$, and the basis weight of the elastic nonwoven fabric in the second layer was change from 10.0 g/cm$^2$ to 18.8 g/cm$^2$.

[Example 5]

The multilayer nonwoven fabric was obtained in the same manner as Example 1 except that the basis weight of each extensible spunbonded nonwoven fabric in the first layer and the third layer and the basis weight of the elastic nonwoven fabric in the second layer were change from 10.0 g/cm$^2$ to 20.0 g/cm$^2$.

[Example 6]

The multilayer nonwoven fabric was obtained in the same manner as Example 2 except that the basis weight of each extensible spunbonded nonwoven fabric in the first layer and the third layer and the basis weight of the elastic nonwoven fabric in the second layer were change from 10.0 g/cm$^2$ to 20.0 g/cm$^2$.

[Comparative Example 2]

The multilayer nonwoven fabric was obtained in the same manner as Comparative Example 1 except that the basis weight of each extensible spunbonded nonwoven fabric in the first layer and the third layer and the basis weight of the elastic nonwoven fabric in the second layer were change from 10.0 g/cm$^2$ to 20.0 g/cm$^2$.

[Comparative Example 3]

The multilayer nonwoven fabric was obtained in the same manner as Comparative Example 2 except that the basis weight of the elastic nonwoven fabric in the second layer was change from 20.0 g/cm$^2$ to 25.0 g/cm$^2$.

-Evaluation-

(Spinnability)

During the production of the nonwoven fabrics of each example, the spinning condition near the nozzle surface of the spunbonded nonwoven fabric producing apparatus was visually observed, and the number of yarn breaks per 5 minutes (unit: times/ 5 min) was counted. When the number of yarn breaks was 0 times/ 5 min, it was evaluated as "A", and when the yarn breakage occurred and the nonwoven fabric could not be collected, it was evaluated as "B" (Tables 1 and 2).

-Evaluation of Stretch Characteristics (Stress at 50% Extending, Stress at 50% Recovering)

Five test pieces of 50 mm (CD) $\times$ 200 mm (MD) were sampled from the multilayer nonwoven fabric of each example using a universal tensile tester (IM-201 type, manufactured by Intesco Co., Ltd.). Subsequently, each of the sampled test pieces was extended by 100% under conditions of a sample width of 50 mm, a chuck distance of 100 mm, and a tensile speed of 100 mm/min, and then immediately recovered to the original length at the same speed. One cycle of this operation was further repeated, and stress in a case in which a stretching ratio became 50% during extending in the second cycle was defined as stress at 50% extending, and stress in a case in which a stretching ratio became 50% during recovering in the second cycle was defined as stress at 50% recovering. Subsequently, the value of [stress at 50% recovering ÷ stress at 50% extending] was measured as the measure of the stretch characteristics, and the average value of the five test pieces was evaluated as stretch characteristics. The larger the value of [stress at 50% recovering ÷ stress at 50% extending] is, the more excellent the stretch characteristics are (Table 1 and Table 2).

In addition, Table 1 and Table 2 show the results of measuring the storage elastic modulus at each temperature, the ratio of the storage elastic modulus, the maximum load elongation, and the basis weight of each nonwoven fabric in each example by the above-mentioned measuring method.

[Table 1]

| | | | | Example 1 | | Example 2 | | Comparative Example 1 | |
|---|---|---|---|---|---|---|---|---|---|
| Composition of Nonwoven Fabric | Outer Layer (Extensible SB Nonwoven Fabric ) | Polymer | Portion | Sheath | Core | Sheath | Core | Sheath | Core |
| | | | Type | PP | PP | PP | PP | PP | PP |
| | | Ratio | [%] | 90 | 10 | 90 | 10 | 90 | 10 |
| | | Maximum Load Elongation | [%] | 155 | | 155 | | 155 | |
| | | Basis Weight | [g/m$^2$] | 10 | | 10 | | 10 | |
| | | Spinnability | [-] | A | | A | | A | |
| | Middle Layer (Elastic Nonwoven Fabric) | Polymer | Type | $\alpha$-olefin copolymer 1A | | $\alpha$-olefin copolymer 1B | | $\alpha$-olefin homopolymer 1C | |
| | | | Carbon Chain | C2/C3 | | C2/C3 | | C3 | |
| | | Basis Weight | [g/m$^2$] | 10 | | 10 | | 10 | |
| | | Spinnability | [-] | A | | A | | A | |
| Property of Elastomer in Elastic Nonwoven Fabric | | Melting Point (low melting point side) | [°C] | 44 | | 56.4 | | 56.1 | |
| | | Degree of Crystallinity | [%] | 9 | | 2.8 | | 14.3 | |
| | | Storage Elastic Modulus E23 | [MPa] | 17.4 | | 12 | | 49.3 | |
| | | Storage Elastic Modulus E40 | [MPa] | 8.77 | | 6.55 | | 17.7 | |
| | | Ratio of Storage Elastic Modulus (E40/E23) | [%] | 50.4 | | 54.6 | | 35.9 | |
| Property of Multilayer Nonwoven Fabric | | Basis Weight | [g/m$^2$] | 30 | | 30 | | 30 | |
| | | Stress at 50% Extending | [N/50mm] | 0.49 | | 0.52 | | 0.83 | |
| | | Stress at 50% Recovering | [N/50mm] | 0.28 | | 0.27 | | 0.36 | |
| | | Stretch Characteristics (Recovering/Extending) | [-] | 0.58 | | 0.53 | | 0.43 | |

[Table 2]

| | | | | Example 3 | | Example 4 | | Example 5 | | Example 6 | | Comparative Example 2 | | Comparative Example 3 | |
|---|---|---|---|---|---|---|---|---|---|---|---|---|---|---|---|
| Composition of Nonwoven Fabric | Outer Layer (Extensible SB Nonwoven Fabric) | Polymer | Portion | Sheath | Core | Sheath | Core | Sheath | Core | Sheath | Core | Sheath | Core | Sheath | Core |
| | | | Type | PP | PP | PP | PP | PP | PP | PP | PP | PP | PP | PP | PP |
| | | Ratio | [%] | 90 | 10 | 90 | 10 | 90 | 10 | 90 | 10 | 90 | 10 | 90 | 10 |
| | | Basis Weight | [g/m²] | 16.7 | | 15.6 | | 20 | | 20 | | 20 | | 20 | |
| | | Spinnability | [-] | A | | A | | A | | A | | A | | A | |
| | Middle Layer (Elastic Nonwoven Fabric) | Polymer | Type | α-olefin copolymer 1A | | α-olefin copolymer 1A | | α-olefin copolymer 1A | | α-olefin copolymer 1B | | α-olefin homopolymer 1C | | α-olefin homopolymer 1C | |
| | | | Carbon Chain | C2/C3 | | C2/C3 | | C2/C3 | | C2/C3 | | C3 | | C3 | |
| | | Basis Weight | [g/m²] | 16.7 | | 18.8 | | 20 | | 20 | | 20 | | 25 | |
| | | Spinnability | [-] | A | | A | | A | | A | | A | | A | |
| Property of Elastomer in Elastic Nonwoven Fabric | | Melting Point (low melting point side) | [°C] | 44 | | 44 | | 44 | | 56.4 | | 56.1 | | 56.1 | |
| | | Degree of Crystallinity | [%] | 9 | | 9 | | 9 | | 2.8 | | 14.3 | | 14.3 | |
| | | Storage Elastic Modulus E23 | [MPa] | 17.4 | | 17.4 | | 17.4 | | 12 | | 49.3 | | 49.3 | |
| | | Storage Elastic Modulus E40 | [MPa] | 8.77 | | 8.77 | | 8.77 | | 6.55 | | 17.7 | | 17.7 | |
| | | Ratio of Storage Elastic Modulus (E40/E23) | [%] | 50.4 | | 50.4 | | 50.4 | | 54.6 | | 35.9 | | 35.9 | |
| Property of Multilayer Nonwoven Fabric | | Total Basis Weight | [g/m²] | 50 | | 50 | | 60 | | 60 | | 60 | | 65 | |
| | | Stress at 50% Extending | [N/50mm] | 1.13 | | 1.39 | | 1.48 | | 0.92 | | 2.28 | | 3.04 | |
| | | Stress at 50% Recovering | [N/50mm] | 0.47 | | 0.66 | | 0.66 | | 0.44 | | 0.83 | | 0.91 | |
| | | Stretch Characteristics (Recovering/Extending) | [-] | 0.41 | | 0.48 | | 0.45 | | 0.48 | | 0.36 | | 0.30 | |

16

As shown in Table 1 and Table 2, it was found that the multilayer nonwoven fabric of the examples was superior to the multilayer nonwoven fabric of the comparative examples in stretch characteristics and a stress retention.

**Claims**

1. A multilayer nonwoven fabric, comprising:

   an elastic nonwoven fabric comprising an $\alpha$-olefin copolymer at a ratio of 90% by mass to 100% by mass with respect to the total amount of the elastic nonwoven fabric, wherein the $\alpha$-olefin copolymer has a ratio of a storage elastic modulus E40 at 40°C and a storage elastic modulus E23 at 23°C (E40/E23) of 37% or more, the storage elastic moduli E23 and E40 being determined according to the method described in the description; and
   an extensible spunbonded nonwoven fabric disposed on at least one side of the elastic nonwoven fabric, wherein the extensible spunbonded nonwoven fabric is formed of an olefin polymer composition comprising an olefin polymer, the extensible spunbonded nonwoven fabric does not include another polymer, i.e. a polymer other than the olefin polymer, or a content of the other polymer in the extensible spunbonded nonwoven fabric is more than 0% by mass and less than 10% by mass with respect to the total of the olefin polymer and the other polymer, and wherein a maximum load elongation of the extensible spunbonded nonwoven fabric in at least one direction is 45% or more, the maximum load elongation being determined according to the method described in the description.

2. The multilayer nonwoven fabric according to claim 1, wherein the storage elastic modulus E23 at 23°C of the $\alpha$-olefin copolymer is 30 MPa or less.

3. The multilayer nonwoven fabric according to claim 1 or 2, wherein the $\alpha$-olefin copolymer comprises a copolymer of ethylene and propylene.

4. The multilayer nonwoven fabric according to any one of claims 1 to 3, wherein a tensile modulus of the $\alpha$-olefin copolymer is 30 MPa or less as obtained by measuring according to the density gradient method of JIS K7161 (2011).

5. The multilayer nonwoven fabric according to any one of claims 1 to 4, wherein the extensible spunbonded nonwoven fabric is disposed on both sides of the elastic nonwoven fabric.

6. The multilayer nonwoven fabric according to any one of claims 1 to 5, wherein the elastic nonwoven fabric is an elastic spunbonded nonwoven fabric.

7. The multilayer nonwoven fabric according to any one of claims 1 to 6, wherein the extensible spunbonded nonwoven fabric is an extensible spunbonded nonwoven fabric consisting of a concentric sheath-core composite fiber having a core portion, formed by a low MFR olefin polymer having MFR in a range of from 1 g/10 min to 1000 g/10 min, and a sheath portion, formed by a high MFR olefin polymer having MFR in a range of from 1 g/10 min to 1000 g/10 min, and a difference in MFR between the low MFR olefin polymer and the high MFR olefin polymer is 1 g/10 min or more, the MFR being measured under the condition of ASTM D1238, 230°C, and a load of 2.16 kg.

8. The multilayer nonwoven fabric according to any one of claims 1 to 7, wherein the extensible spunbonded nonwoven fabric comprises an olefin polymer composition comprising a crystalline propylene polymer of from 80% by mass to 99% by mass and a high density polyethylene of from 1% by mass to 20% by mass.

9. The multilayer nonwoven fabric according to any one of claims 1 to 8, wherein a ratio (elastic nonwoven fabric:extensible spunbonded nonwoven fabric) of a basis weight of the elastic nonwoven fabric and the extensible spunbonded nonwoven fabric is from 10:90 to 90: 10, the basis weight being determined according to the method described in the description.

10. A stretchable multilayer nonwoven fabric comprising a stretched product of the multilayer nonwoven fabric according to any one of claims 1 to 9, as defined in the description.

11. A fiber product comprising the multilayer nonwoven fabric according to any one of claims 1 to 9 or the stretchable multilayer nonwoven fabric according to claim 10, wherein the fiber product is an absorbent article, a hygiene article, a medical article, a clothing material, or a packaging material.

12. The fiber product of claim 11, which is an absorbent article.

13. The fiber product of claim 11, which is a sanitary mask.

**Patentansprüche**

1. Mehrschichtiger Vliesstoff, umfassend:

einen elastischen Vliesstoff, der ein α-Olefin-Copolymer in einem Massenanteil von 90 Masse-% bis 100 Masse-% bezogen auf die Gesamtmenge des elastischen Vliesstoffs umfasst, wobei das α-Olefin-Copolymer ein Verhältnis des Speicherelastizitätsmoduls E40 bei 40 °C zum Speicherelastizitätsmodul E23 bei 23 °C (E40/E23) von 37 % oder mehr aufweist, wobei die Speicherelastizitätsmoduln E23 und E40 nach dem in der Beschreibung beschriebenen Verfahren bestimmt werden; und

einen dehnbaren Spinnvliesstoff, der auf mindestens einer Seite des elastischen Vliesstoffs angeordnet ist, wobei der dehnbare Spinnvliesstoff aus einer Olefinpolymerzusammensetzung gebildet ist, die ein Olefinpolymer umfasst, wobei der dehnbare Spinnvliesstoff kein weiteres Polymer einschließt, d. h. kein vom Olefinpolymer verschiedenes Polymer, oder ein Gehalt des anderen Polymers im dehnbaren Spinnvliesstoff mehr als 0 Masse-% und weniger als 10 Masse-% bezogen auf die Gesamtmenge des Olefinpolymers und des anderen Polymers beträgt, und wobei eine Bruchdehnung bei Maximallast des dehnbaren Spinnvliesstoffs in mindestens einer Richtung 45 % oder mehr beträgt, wobei die Bruchdehnung bei Maximallast nach dem in der Beschreibung beschriebenen Verfahren bestimmt wird.

2. Mehrschichtiger Vliesstoff nach Anspruch 1, wobei das Speicherelastizitätsmodul E23 bei 23 °C des α-Olefin-Copolymers 30 MPa oder weniger beträgt.

3. Mehrschichtiger Vliesstoff nach Anspruch 1 oder 2, wobei das α-Olefin-Copolymer ein Copolymer aus Ethylen und Propylen umfasst.

4. Mehrschichtiger Vliesstoff nach einem der Ansprüche 1 bis 3, wobei ein Zugmodul des α-Olefin-Copolymers 30 MPa oder weniger beträgt, bestimmt gemäß dem Dichtegradientenverfahren nach JIS K7161 (2011).

5. Mehrschichtiger Vliesstoff nach einem der Ansprüche 1 bis 4, wobei der dehnbare Spinnvliesstoff auf beiden Seiten des elastischen Vliesstoffs angeordnet ist.

6. Mehrschichtiger Vliesstoff nach einem der Ansprüche 1 bis 5, wobei der elastische Vliesstoff ein elastischer Spinnvliesstoff ist.

7. Mehrschichtiger Vliesstoff nach einem der Ansprüche 1 bis 6, wobei der dehnbare Spinnvliesstoff ein dehnbarer Spinnvliesstoff ist, der aus einer konzentrischen Mantel-Kern-Verbundfaser besteht, die einen Kernbereich aufweist, gebildet aus einem Olefinpolymer mit niedrigem MFR, das einen MFR im Bereich von 1 g/10 min bis 1000 g/10 min aufweist, und einem Mantelbereich, gebildet aus einem Olefinpolymer mit hohem MFR, das einen MFR im Bereich von 1 g/10 min bis 1000 g/10 min aufweist, und eine Differenz im MFR zwischen dem Olefinpolymer mit niedrigem MFR und dem Olefinpolymer mit hohem MFR beträgt 1 g/10 min oder mehr, wobei der MFR unter den Bedingungen von ASTM D1238 bei 230 °C und einer Last von 2,16 kg gemessen wird.

8. Mehrschichtiger Vliesstoff nach einem der Ansprüche 1 bis 7, wobei der dehnbare Spinnvliesstoff eine Olefinpolymerzusammensetzung umfasst, die ein kristallines Propylenpolymer in einem Massenanteil von 80 Masse-% bis 99 Masse-% und ein hochdichtes Polyethylen in einem Massenanteil von 1 Masse-% bis 20 Masse-% umfasst.

9. Mehrschichtiger Vliesstoff nach einem der Ansprüche 1 bis 8, wobei ein Verhältnis (elastischer Vliesstoff:dehnbarer Spinnvliesstoff) des Flächengewichts des elastischen Vliesstoffs und des dehnbaren Spinnvliesstoffs von 10:90 bis 90:10 beträgt, wobei das Flächengewicht nach dem in der Beschreibung beschriebenen Verfahren bestimmt wird.

10. Streckbarer mehrschichtiger Vliesstoff, umfassend ein Streckprodukt des mehrschichtigen Vliesstoffs nach einem der Ansprüche 1 bis 9, wie in der Beschreibung definiert.

11. Faserprodukt, umfassend den mehrschichtigen Vliesstoff nach einem der Ansprüche 1 bis 9 oder den streckbaren mehrschichtigen Vliesstoff nach Anspruch 10, wobei das Faserprodukt ein absorbierender Artikel, ein Hygieneartikel, ein medizinischer Artikel, ein Bekleidungsmaterial oder ein Verpackungsmaterial ist.

**12.** Faserprodukt nach Anspruch 11, das ein absorbierender Artikel ist.

**13.** Faserprodukt nach Anspruch 11, das eine Hygienemaske ist.


**Revendications**

**1.** Tissu non tissé multicouche, comprenant :

un tissu non tissé élastique comprenant un copolymère d'$\alpha$-oléfine à un rapport de 90 % en masse à 100 % en masse par rapport à la quantité totale du tissu non tissé élastique, dans lequel le copolymère d'$\alpha$-oléfine présente un rapport (E40/E23) d'un module d'élasticité de stockage E40 à 40 °C sur un module d'élasticité de stockage E23 à 23 °C supérieur ou égal à 37 %, les modules d'élasticité de stockage E23 et E40 étant déterminés selon le procédé décrit dans la description ; et
un tissu non tissé filé-lié extensible disposé sur au moins un côté du tissu non tissé élastique, dans lequel le tissu non tissé filé-lié extensible est formé d'une composition de polymère oléfinique comprenant un polymère oléfinique, le tissu non tissé filé-lié extensible n'inclut pas d'autre polymère, c'est-à-dire de polymère autre que le polymère oléfinique, ou une teneur de l'autre polymère dans le tissu non tissé filé-lié extensible est supérieure à 0 % en masse et inférieure à 10 % en masse par rapport au total du polymère oléfinique et de l'autre polymère, et dans lequel un allongement à la charge maximale du tissu non tissé filé-lié extensible dans au moins une direction est supérieur ou égal à 45 %, l'allongement à la charge maximale étant déterminé selon le procédé décrit dans la description.

**2.** Tissu non tissé multicouche selon la revendication 1, dans lequel le module d'élasticité de stockage E23 à 23 °C du copolymère d'$\alpha$-oléfine est inférieur ou égal à 30 MPa.

**3.** Tissu non tissé multicouche selon la revendication 1 ou la revendication 2, dans lequel le copolymère d'$\alpha$-oléfine comprend un copolymère d'éthylène et de propylène.

**4.** Tissu non tissé multicouche selon l'une quelconque des revendications 1 à 3, dans lequel un module de traction du copolymère d'$\alpha$-oléfine est inférieur ou égal à 30 MPa tel qu'obtenu par mesure selon la méthode par gradient de densité conformément à la norme JIS K7161 (2011).

**5.** Tissu non tissé multicouche selon l'une quelconque des revendications 1 à 4, dans lequel le tissu non tissé filé-lié extensible est disposé sur les deux côtés du tissu non tissé élastique.

**6.** Tissu non tissé multicouche selon l'une quelconque des revendications 1 à 5, dans lequel le tissu non tissé élastique est un tissu non tissé filé-lié élastique.

**7.** Tissu non tissé multicouche selon l'une quelconque des revendications 1 à 6, dans lequel le tissu non tissé filé-lié extensible est un tissu non tissé filé-lié extensible consistant en une fibre composite concentrique gaine-noyau présentant une partie noyau, formée par un polymère oléfinique à faible indice de fluidité MFR compris dans une plage allant de 1 g/10 min à 1 000 g/10 min, et une partie gaine, formée par un polymère oléfinique à indice de fluidité MFR élevé compris dans une plage allant de 1 g/10 min à 1 000 g/10 min, et une différence de MFR entre le polymère oléfinique à indice de fluidité MFR faible et le polymère oléfinique à indice de fluidité MFR élevé est supérieure ou égale à 1 g/10 min, le MFR étant mesuré dans les conditions de la norme ASTM D1238, à 230 °C, sous une charge de 2,16 kg.

**8.** Tissu non tissé multicouche selon l'une quelconque des revendications 1 à 7, dans lequel le tissu non tissé filé-lié extensible comprend une composition de polymère oléfinique comprenant un polymère de propylène cristallin compris de 80 % en masse à 99 % en masse et un polyéthylène de haute densité compris de 1 % en masse à 20 % en masse.

**9.** Tissu non tissé multicouche selon l'une quelconque des revendications 1 à 8, dans lequel un rapport (tissu non tissé élastique : tissu non tissé filé-lié extensible) d'une masse surfacique du tissu non tissé élastique et du tissu non tissé filé-lié extensible est compris de 10:90 à 90:10, la masse surfacique étant déterminée selon la méthode décrite dans la description.

10. Tissu non tissé multicouche extensible étirable comprenant un produit étiré du tissu non tissé multicouche selon l'une quelconque des revendications 1 à 9, tel que défini dans la description.

11. Produit fibreux comprenant le tissu non tissé multicouche selon l'une quelconque des revendications 1 à 9 ou le tissu non tissé multicouche étirable selon la revendication 10, dans lequel le produit fibreux est un article absorbant, un article d'hygiène, un article médical, un matériau vestimentaire ou un matériau d'emballage.

12. Produit fibreux selon la revendication 11, qui est un article absorbant.

13. Produit fibreux selon la revendication 11, qui est un masque sanitaire.

FIG. 1

**REFERENCES CITED IN THE DESCRIPTION**

**Patent documents cited in the description**

- JP H07503502 A
- JP 2009062667 A
- JP 2009079341 A
- JP 2007138733 A
- JP 2018201247 A
- JP 2017197890 A
- WO 2016143834 A